# EUROPEAN PATENT APPLICATION

(11) **EP 2 720 238 A1**
(43) Date of publication of application: **16.04.2014**
(21) Application number: 12188026.4
(22) Date of filing: 10.10.2012
(51) Int. Cl.: H01G 9/20, C07F 15/00

(54) **Dye-sensitized solar cells, metal complex dyes therefor, and methods for making such dyes**

(71) Applicant: Solvay SA, 1120 Bruxelles (BE)
(72) Inventor: Braun, Max Josef, 30900 WEDEMARK (DE); Jung, Il, 1024 ECUBLENS (CH)
(74) Representative: Mross, Stefan P.M.

(57) **Abstract**

Provided are dye-sensitized solar cells, comprising at least one substituted monopyridine ligand or polypyridine ligand, as a sensitizing-dye, and a cobalt complex, as a redox mediator, wherein the substituted monopyridine ligand and/or polypyridine ligand comprises at least one substituted aryl group. The present invention also concerns metal complex dyes therefor, and methods for making such dyes.

## Description

### TECHNICAL FIELD

The present invention relates to dye-sensitized solar cells comprising specific metal complex as a sensitizing-dye and cobalt complex as a redox mediator, metal complex dyes therefor, and methods for making such dyes.

### BACKGROUND OF THE INVENTION

Conventional solar cells convert light into electricity by exploiting the photovoltaic effect that exists at semiconductor junctions. In other words, the commercial solar cells absorb energy from visible light and convert excited charge carriers thereof to electric energy. At present, the main commercial solar cells are silicon-based solar cells. For the silicon-based solar cell, there are shortcomings in that high energy costs for material processing is required and many problems to be addressed such as environmental burdens and cost and material supply limitations are involved. For amorphous silicon solar cells, there are also shortcomings in that energy conversion efficiency decreases when used for a long time, due to deterioration in a short period.

One of the low-cost organic solar cells is a dye-sensitized solar cell (DSSC) which is formed based on a semiconductor sensitized by a dye to absorb incoming light to produce excited electron, an electrode connected to the semiconductor, a counter electrode and an electrolyte usually comprising the triiodide/iodide (I₃⁻/I⁻) redox mediator.

The triiodide/iodide redox mediator has several drawbacks from both scientific and commercial point of views. US 7,019,138 B2 discloses certain cobalt complexes as a candidate of alternative redox mediator.

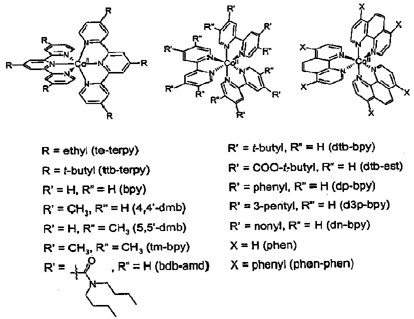

In spite of their advantages, however, the cobalt complex based redox mediators generally have poor compatibility with sensitizing-dye molecules having metal complex structure, such as N719.

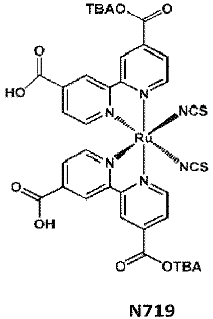

### DESCRIPTION OF THE INVENTION

The purpose of the present invention is to provide metal complex dyes which show superior compatibility with the cobalt complex based redox mediator. Another purpose of the present invention is to provide dye-sensitized solar cells based on a combination of metal complex dye and cobalt complex based redox mediator, which has advantageous performance.

The present invention therefore relates to dye-sensitized solar cells, comprising a metal complex comprising at least one substituted monopyridine ligand or polypyridine ligand, as a sensitizing-dye, and a cobalt complex, as a redox mediator, wherein the substituted monopyridine ligand and/or polypyridine ligand comprises at least one substituted aryl group.

It has been surprisingly found by the present inventors that the metal complex comprising at least one substituted monopyridine ligand or polypyridine ligand shows superior compatibility with cobalt complex based redox mediator when certain substituent(s) is introduced into the substituted monopyridine ligand and/or polypyridine ligand of the metal complex. This advantage allows the use of cobalt complex as redox mediator in DSSC system in which metal complex dye molecule is adopted as a sensitizing-dye, without causing a significant decrease in the performance of the system. The metal complex dyes usually have better total performance, such as in terms of efficiency and/or stability, than other type of dyes, such as organic dyes having D-π-A structure (wherein D is donor group, π is bridge group and A is acceptor group), or macrocyclic dyes, for example porphyrin dyes and phthalocyanine dyes.

However, it has been understood that the conventional metal complex dyes have poor compatibility with cobalt complex based redox mediator, despite of the advantages of using such redox mediator. Such advantages of cobalt complex based redox mediator over triiodide/iodide system include noncorrosive characteristic, high transmittance (and colorless), and high Voc. The present inventors have found that certain metal complex dyes can be combined with cobalt complex based redox mediator.

In the present invention, "redox mediator" is understood to denote in particular molecules or ions which mediate electron transfer from a counter electrode to an oxidized dye by their reversible reduction-oxidation function.

In the present invention, "alkyl groups" is understood to denote in particular a straight chain, branched chain, or cyclic hydrocarbon groups usually having from 1 to 20 carbon atoms. Examples of alkyl groups include methyl, ethyl, propyl, isopropyl, butyl, isobutyl, sec-butyl, tert-butyl, pentyl, neopentyl, hexyl, cyclopropyl, cyclobutyl, cyclopentyl, and cyclohexyl.

In the present invention, "alkoxy groups" is understood to denote in particular a straight chain, branched chain, or cyclic hydrocarbon groups usually having from 1 to 20 carbon atoms singularly bonded to oxygen (Alk-O-).

In the present invention, "aryl groups" is understood to denote in particular any functional group or substituent derived from an aromatic ring. In particular, the aryl groups can have 6 to 20 carbon atoms (preferably 6 to 12 due to its easiness of synthesis at a low cost) in which some or all of the hydrogen atoms of the aryl group may or may not be substituted with other groups, especially alkyl groups, alkoxy groups, aryl groups, aryloxy groups, thioalkoxy groups, heterocycles, amino groups or hydroxyl groups. The aryl groups are preferably optionally substituted phenyl groups, naphthyl groups, anthryl group and phenanthryl group.

In the present invention, "aryloxy groups" is understood to denote in particular the aryl group as defined above singularly bonded to oxygen (Ar-O-).

In the present invention, "heterocycles" is understood to denote in particular a cyclic compound, which has at least one heteroatom as a member of its one or more rings. Frequent heteroatoms within the ring include sulfur, oxygen and nitrogen. The heterocycles can be either saturated or unsaturated, and may be 3-membered, 4-membered, 5-membered, 6-membered or 7-membered ring. The heterocycles can be further fused with other one or more ring systems. Examples of the heterocycles include pyrrolidines, oxolanes, thiolanes, pyrroles, furans, thiophenes, piperidines, oxanes, thianes, pyridines, pyrans, pyrazoles, imidazoles, and thiopyrans, and their derivatives. The heterocycles can further be substituted by other groups, such as alkyl groups, alkoxy groups, aryl groups, thioalkoxy groups, amino groups or aryloxy groups as defined above.

In the present invention, "amino groups" is understood to denote in particular aliphatic amines or aromatic amines and encompass compounds comprising one or multiple amino groups. The amino groups can be primary amines, secondary amines and tertiary amines, wherein one or more hydrogen atoms may or may not be substituted with other groups, such as alkyl groups or aryl groups.

In the present invention, "halogenated" is understood to denote in particular at least one of the hydrogen atoms of the following chemical group has been replaced by a halogen atom, preferably selected from fluorine and chlorine, more preferably fluorine. If all of the hydrogen atoms have been replaced by halogen atoms, the halogenated chemical group is perhalogenated. For instance, "halogenated alkyl groups" include (per)fluorinated alkyl groups such as (per)fluorinated methyl, ethyl, propyl, isopropyl, butyl, isobutyl, *sec*-butyl or *tert*-butyl; and for instance -CF₃, -C₂F₅, heptafluoroisopropyl (-CF(CF₃)₂), hexafluoroisopropyl (-CH(CF₃)₂) or -CF₂(CF₂)₄CF₃. Non-limiting example of "halogenated aryl groups" include -C₆F₅.

In the present invention, "anchoring groups" is understood to denote in particular groups that will allow attachment of the dyes onto a semiconductor, such as TiO₂ and TiOF₂. Suitable anchoring groups are for instance selected from, but not limited to, the group consisting of -COOH, -PO₃H₂, - P(=O)(Ra)(OH) (wherein Ra is selected from the groups consisting of optionally branched C1-C18 alkyl groups and optionally substituted C5-C12 aryl groups, and halogenated derivatives thereof), -PO₄H₂, -PO₂HRb (wherein Rb is selected from alkyl groups or aryl groups), -SO₃H, -CONHOH, -NO₂, acetylacetonate, acrylic acid derivatives, malonic acid derivatives, rhodanine-3-acetic acid, propionic acid, deprotonated forms of the aforementioned groups, salts of said deprotonated forms, and chelating groups with π-conducting character. More preferred anchoring groups are -COOH or the salts of deprotonated -COOH. The acrylic acid derivatives may for instance be selected from groups of formula -CH=C(Rc)-COOH where Rc is selected from H, CN, -COOH and optionally halogenated alkyl groups, preferably from H, CN, -COOH and CF₃. The malonic acid derivatives suitable as anchoring groups may for example be selected from groups of formula -CRd=C(COOH)₂ where Rd is selected from H and optionally halogenated alkyl groups, especially from H and optionally fluorinated alkyl groups. Especially preferable salts of -COOH are for instance ammonium salts, or alkali metal salts, more preferably -COOTBA (wherein, TBA indicates tetrabutylammonium).

In one embodiment according to the present invention, the substituted monopyridine ligand and/or polypyridine ligand in the metal complex as a sensitizing-dye is selected from any one of the structures indicated below, which structure can be further substituted:

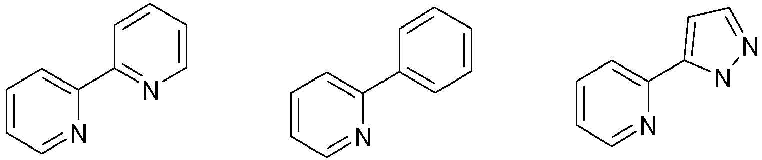

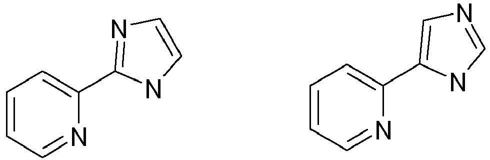

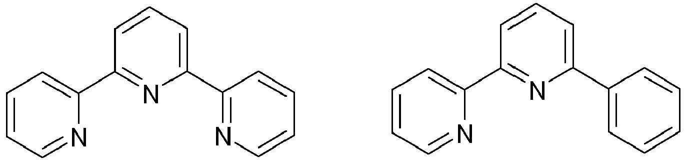

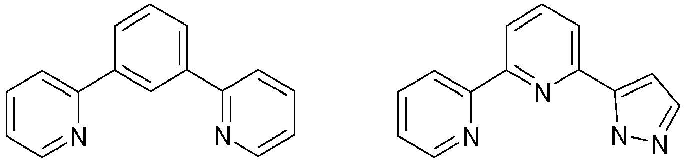

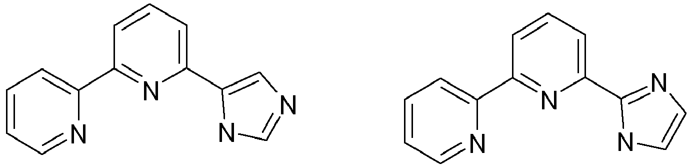

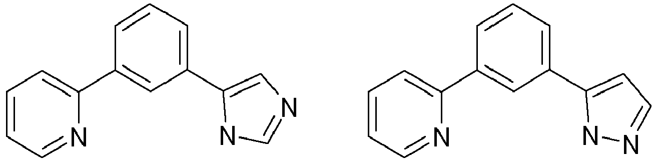

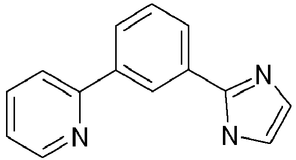

In a further embodiment according to the present invention, the substituted aryl group comprised in the substituted monopyridine ligand and/or polypyridine ligand for the metal complex as a sensitizing-dye is selected from alkoxy or thioalkyl phenyl groups. Preferably, such alkoxy groups or thioalkyl groups contain 2 or more carbon atoms, preferably from 4 to 16 carbon atoms.

In another embodiment according to the present invention, the substituted aryl group comprised in the substituted monopyridine ligand and/or polypyridine ligand for the metal complex as a sensitizing-dye is connected to the substituted monopyridine ligand and/or polypyridine ligand through a bridge selected from the group consisting of aromatic amine groups, heteroatoms, such as sulfur atom (S) and oxygen atom (O), alkenyl groups, aryl groups, and heterocycles, such as thiophene, oligothiophenes, for example dithiophenes and trithiophenes, ethylenedioxythiophenes (EDOT), oligo-EDOTs, cyclopentanedithiophenes (CPDT), and any combination thereof, preferably sulfur atom and thiophene. It has also been surprisingly found that this embodiment has a further advantage in that the elongated ligand according to this embodiment can contribute to enhancement of the exciton coefficient, which results in an increase of efficiency of the system in addition to the superior compatibility with cobalt complex redox mediator.

In a special embodiment according to the present invention, the cobalt complex has the formula (A) :

[L¹ₐCoL²_{b}](X_{c}) (A)

wherein Co is cobalt; a and b are independently integers from 0 to 6, c is an integer from 0 to 3; each X is independently a counter-anion selected from borates or phosphates; and each L¹ is independently selected from the group consisting of unsubstituted or substituted bipyridine ligands and unsubstituted or substituted terpyridine ligands, and each L² is independently a co-ligand. In the present invention, the co-ligand (L²), in case it exists in the cobalt complex, is selected from the group consisting of Cl-, Br-, I-, CN-, NCO-, NCS-, NCSe-, amino groups, phosphate groups, ClO₄⁻, PF₆⁻, borates such as [BF₄]⁻, tetracyanoborate ([B(CN)₄]⁻), [B(Ph)₄]⁻, [B(C₆F₅)₄]⁻, bis(trifluoromethanesulfonyl)imide (TFSI⁻), bis(fluorosulfonyl)imide (FSI⁻), and any combination thereof, preferably ClO₄⁻, PF₆⁻, [BF₄]⁻, ([B(CN)₄]⁻), TFSI⁻ or FSI⁻.

In a further specific embodiment according to the present invention, L¹ in the formula (A) is selected from the group consisting of the following ligands. In this embodiment, a is preferably 2 or 3. 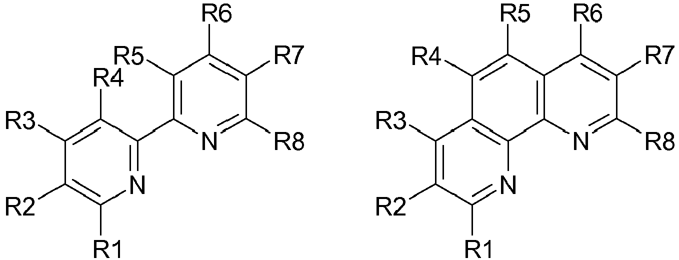 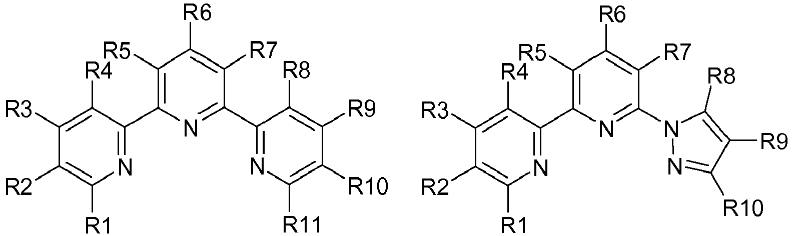 wherein R1, R2, R3, R4, R5, R6, R7, R8, R9, R10 and R11 are independently selected from the group consisting of hydrogen, halogens, in particular fluorine, amino groups, NO₂, CN, alkyl groups, halogenated alkyl groups, aryl groups, halogenated aryl groups, particularly hydrogen, fluorine, alkyl groups, and fluorinated alkyl groups, more particularly hydrogen, fluorine, methyl group, ethyl group, partially or per-fluorinated methyl group, and partially or per-fluorinated ethyl groups.

The cobalt complexes described herein are especially suitable for use in photoelectric conversion devices, particularly in dye-sensitized solar cells.

In a sense of the present invention, the metal complex suitable for the use as a sensitizing-dye in DSSC can be preferably selected from any one of the structures indicated below, which structures can be further substituted, but not limited to them: 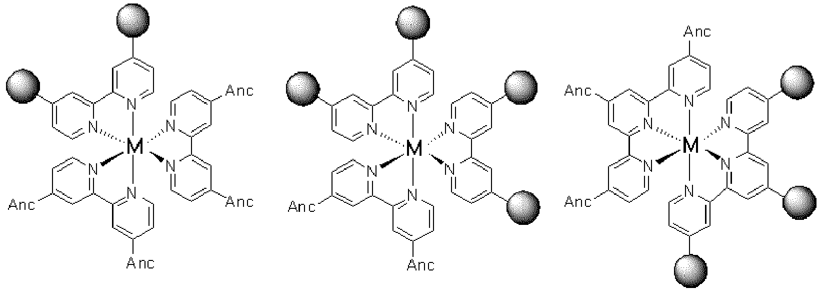 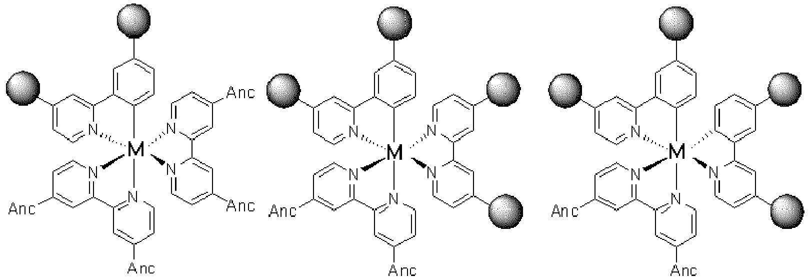 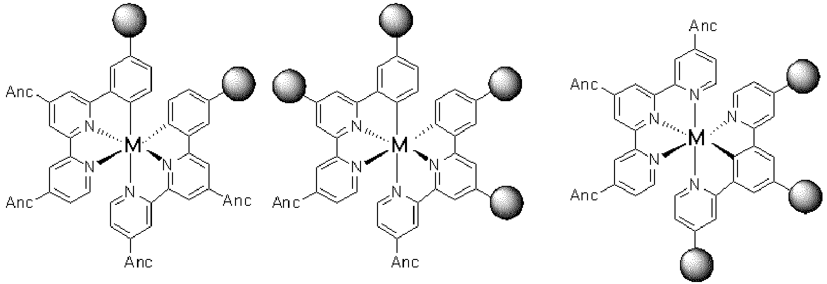 wherein 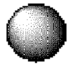 represents the substituted aryl group comprised in the substituted monopyridine ligand and/or polypyridine ligand for the metal complex as a sensitizing-dye, optionally comprising the bridge as denoted in the above, M represents a metal belonging to Group 6, 8, 9, 10 or 11 of the long-format Periodic Table, preferably ruthenium, and Anc is independently selected from anchoring groups.

In a specific embodiment of the present invention, the metal complex suitable for the use as a sensitizing-dye in DSSC has the formula (I):

ML1L2X1X2 (I)

wherein M represents a metal belonging to Group 6, 8, 9, 10 or 11 of the long-format Periodic Table, preferably ruthenium; X1 and X2 are independently selected from the group consisting of halide, -OH, -CN, -SCN, -NCS, and -NCO, preferably -NCS; and L1 and L2 are independently selected from bipyridine ligands, in which at least one of L1 and L2 correspond to the formula (B) or the formula (II) below: 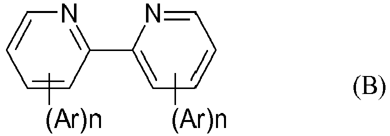 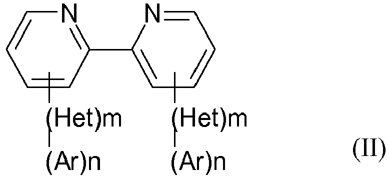 wherein Het is independently selected from a bridge selected from the group consisting of aromatic amine groups, heteroatoms, alkenyl groups, aryl groups, and heterocycles, in particular thiophene, oligothiophenes, ethylenedioxythiophenes (EDOT), oligo-EDOTs, cyclopentanedithiophenes (CPDT), and any combination thereof, preferably sulfur and thiophene, Ar is independently selected from substituted aryl groups, m is independently 1, 2, 3, or 4, and n is 1, 2, 3, or 4.

In an especial embodiment of the present invention, the formula (B) has the formula (B-1), (B-2), (B-3) or (B-4) below: 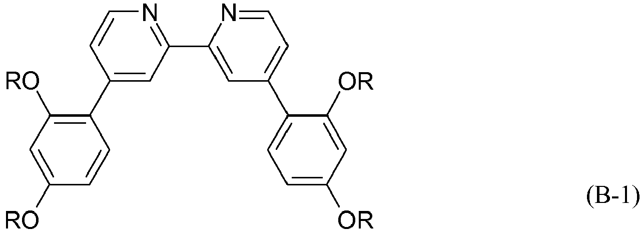 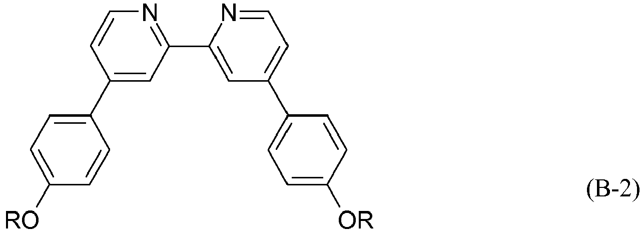 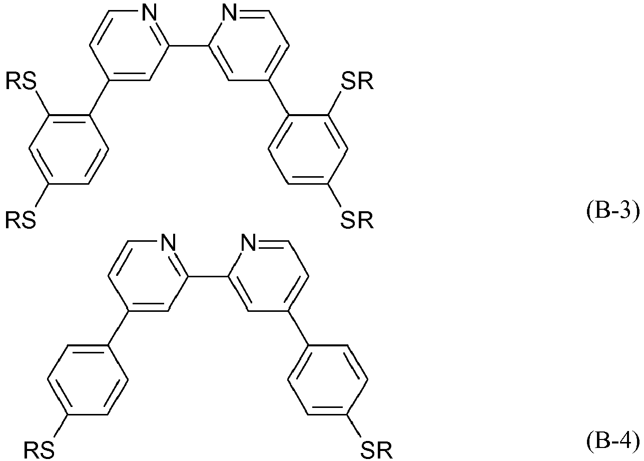 wherein R is selected from alkyl groups, preferably butyl, pentyl, hexyl, heptyl octyl, nonyl or decyl group, especially hexyl group.

As example, the above ligand of the formula (B-1) can be prepared by the reaction scheme below:

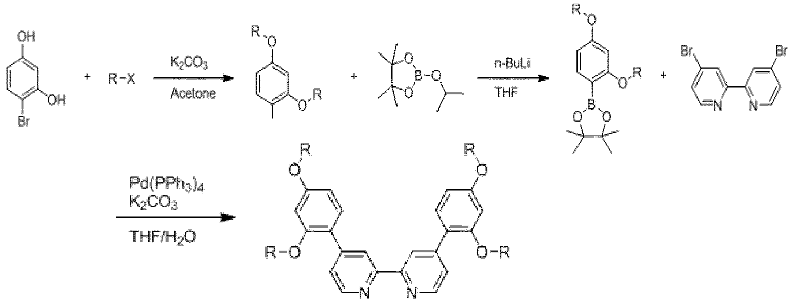

In said reaction scheme, -O-R can be replaced by -S-R. Also, -O-R group in ortho-position can be replaced by hydrogen, while -O-R or -S-R group exists in para-position.

In a more specific embodiment of the present invention, L1 in the formula (I) has the formula (B) or the formula (II) and L2 in the formula (I) has the formula (III) below: 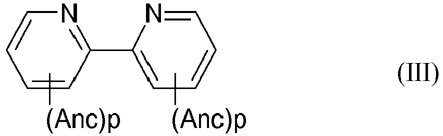 wherein Anc is independently selected from anchoring groups, and p is independently 1, 2, 3 or 4, preferably 1. Preferred examples of the formula (III) are as follows:

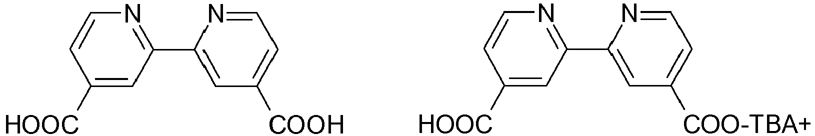

In a very especial embodiment of the present invention, the formula (II) can be preferably selected from any one of the structures indicated below, which structures can be further substituted, but not limited to them: 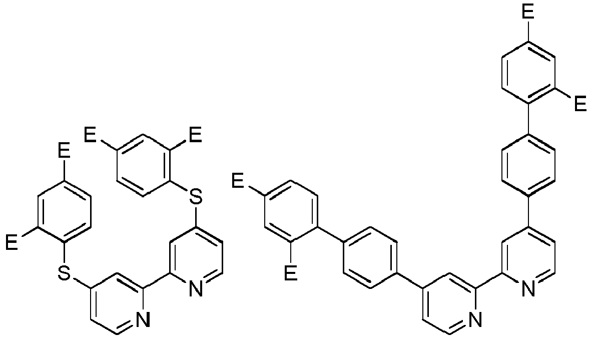 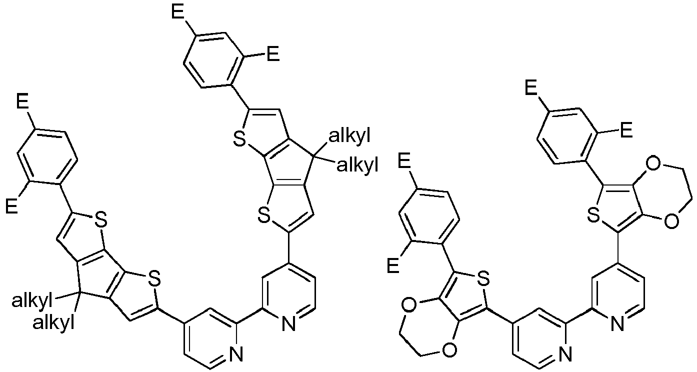 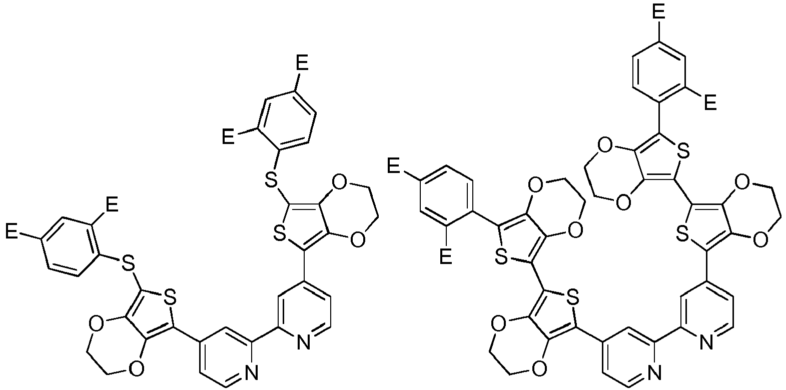 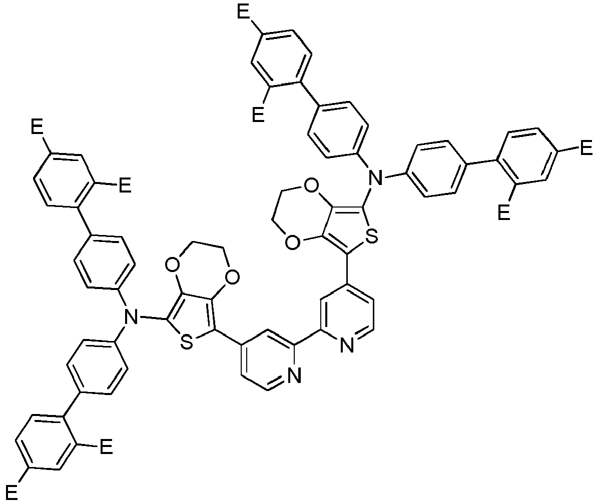 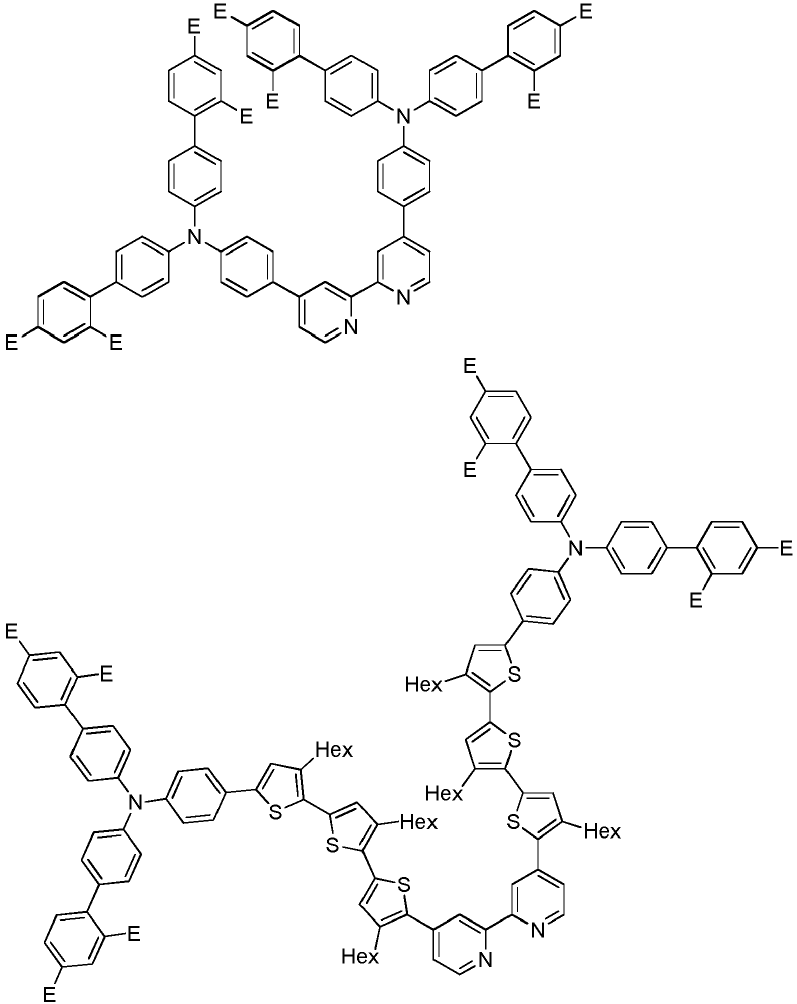 wherein E stands for hydrogen, -OR or -SR (where R is selected from alkyl groups, preferably butyl, pentyl, hexyl, heptyl, octyl, nonyl or decyl group) provided at least one of E is -OR or -SR, preferably both E are -OR or -SR, or E in ortho-position can be hydrogen while E in para-position is -OR or -SR, and Hex represents hexyl group.

In a very specific embodiment of the present invention, the metal complex suitable for the use as a sensitizing-dye in DSSC has at least one ligand of formula (II-1) below: 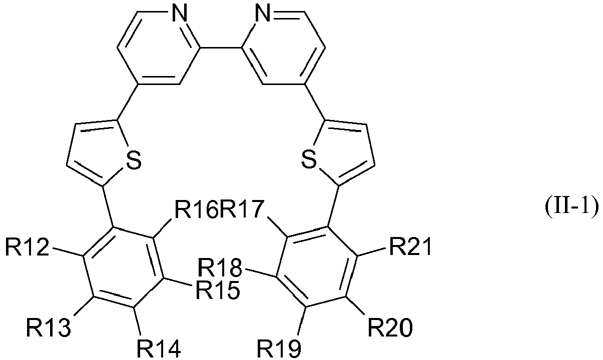 wherein R12, R13, R14, R15, R16, R17, R18, R19, R20 and R21 are independently selected from the group consisting of hydrogen, halogens, cyano, alkyl groups, halogenated alkyl groups, alkoxy groups, halogenated alkoxy groups, aryl groups, halogenated aryl groups, aryloxy groups, halogenated aryloxy groups, heterocycles, halogenated heterocycles, heterocyclic alkyl groups, halogenated heterocyclic alkyl groups, heterocyclic alkoxy groups, halogenated heterocyclic alkoxy groups, heterocyclic aryl groups, halogenated heterocyclic aryl groups, heterocyclic aryloxy groups, halogenated heterocyclic aryloxy groups, amino groups, halogenated amino groups, and NO₂. Preferably, R12, R14, R19 and R21, or R14 and R19 are alkoxy groups or thioalkyl groups, while the rest are hydrogen. Preferred examples of alkoxy groups or thioalkyl groups include butyloxy, pentyloxy, hexyloxy, heptyloxy, octyloxy, nonyloxy and decyloxy group, thiobutyl, thiopentyl, thiohexyl, thioheptyl, thiooctyl, thiononyl and thiodecyl group, especially hexyloxy group.

In a further specific embodiment of the present invention, the metal complex suitable for the use as a sensitizing-dye in DSSC has any one of the formulae (I-1), (I-2), (I-3), (I-4) and (I-5) below: 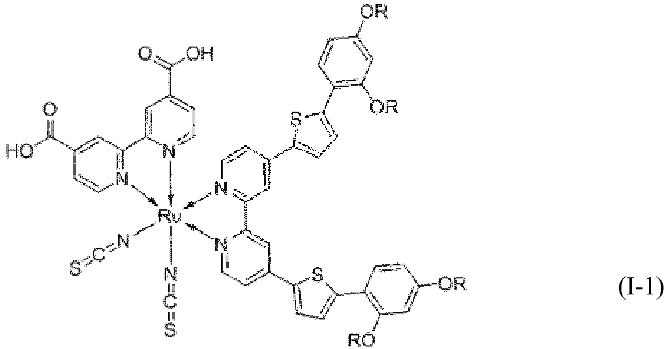 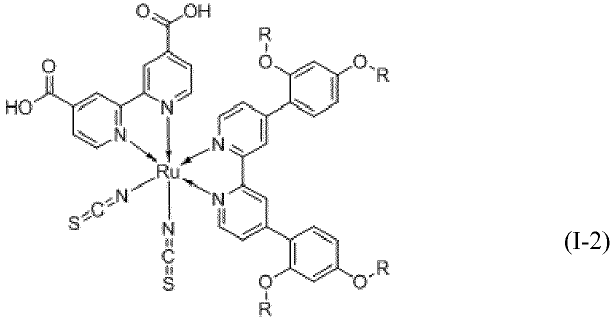 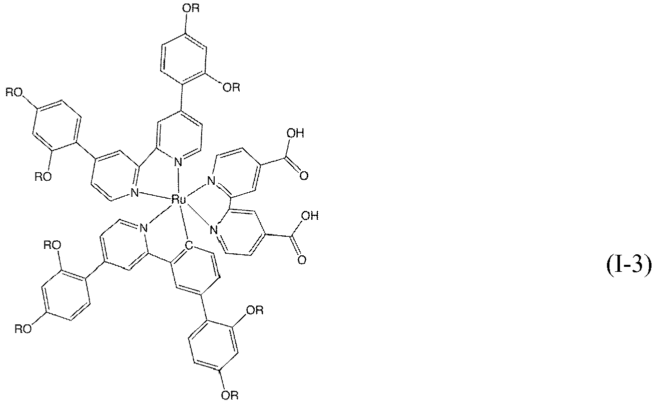 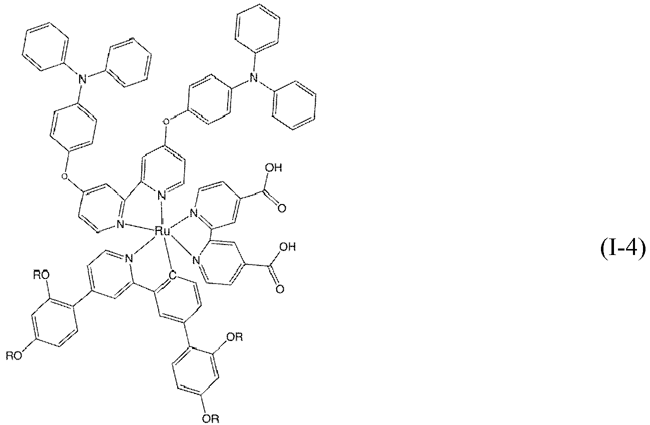 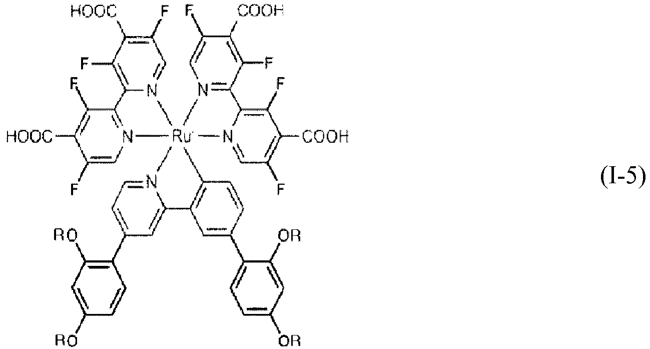 wherein R is alkyl groups, particularly hexyl group.

The basic element of a DSSC is generally a semiconductor, such as TiO₂ (titanium dioxide) nanoparticulate structure sensitized with dye molecules to form the core of a DSSC. The assembly of titanium dioxide nanoparticles is well connected to their neighbors. TiO₂ is the preferred material for the nanoparticles since its surface is highly resistant to the continued electron transfer. However, TiO₂ only absorbs a small fraction of the solar photons (those in the UV). The dye molecules attached to the semiconductor surface are used to harvest a great portion of the solar light. Another key component of the DSSC is an electrolyte which transports positive charge carriers from the sensitizing-dye to the back contact of the device. Electrolytes containing the iodide/triiodide redox system are most commonly used. However, the iodide/triiodide redox system suffers from the disadvantages, such as low redox potential, corrosive characteristic to metals, and competition with the sensitizing-dye in absorbing light. In this context, the cobalt complex described herein can work in DSSC as redox mediator having improved characteristics, compared to traditional iodide/triiodide redox system. In the present invention, such cobalt complex is used in combination with the metal complex sensitizing-dye described in the present invention without causing a significant decrease in the performance of the system.

The metal complex dye compounds usually consist of one metal atom and organic moiety that provides the required properties (e.g., wide absorption range, fast electron injection, and stability). The dye is sensible to the visible light. The light excites electrons from highest occupied molecular orbitals (HOMO) to lowest unoccupied molecular orbitals (LUMO), which is injected to the semiconductor particles (usually TiO₂). The particulate semiconductor functions as the transporter of light-induced electrons towards the external contact, a transparent conductor that lies at the basis of the semiconductor (usually TiO₂) film.

Construction of a DSSC would be well known by a person skilled in the art. Generally, the DSSC comprises an electrode connected to semiconductor, a counter electrode, and an electrolyte. The electrode connected to semiconductor and the counter electrode are arranged in a sandwich-like configuration, and the electrolyte is inserted between the two electrodes.

The material for the counter electrode is not limited as long as the cathode is formed from a material having conductivity. For a non-limiting example, a substrate comprising electrically conductive transparent glass which contains a small amount of platinum or conductive carbon adhering to the surface can be suitably used. As the electrically conductive transparent glass, a glass made of tin oxide or indium-tin oxide (ITO) can be used.

The electrode connected to semiconductor has a substrate made of electrically conductive transparent glass and a semiconducting layer comprising a semiconductor and the sensitizing-dye compound described in the present invention adsorbed thereto. As an example of the electrically conductive transparent glass for such electrode, a glass comprising the materials described above for the cathode can be used, but not limited to them.

As non-limiting examples of materials for the semiconductor, metal oxides such as titanium oxides, niobium oxides, zinc oxides, tin oxides, tungsten oxides, and indium oxides, preferably TiO₂ and SnO₂ are included. TiOF₂ (titanyl oxyfluoride, titanium oxyfluoride or titanium fluoride oxide) can also be envisaged as a suitable semiconductor. TiOF₂ might be suitable when combined with sensitizing-dyes and/or cobalt complex redox mediator of the present invention. The materials may be used as the sole semiconductor in the DSSC semiconductor layer or may be combined in mixture with any other suitable semiconductor layer material such as gallium oxide etc.

In one aspect of the manufacture of the DSSC, the metal complex dye compound of the present invention is usually caused to be adsorbed on the semiconductor. The dye can be adsorbed, for example, by causing the electrode connected to semiconductor comprising substrate of electrically conductive transparent glass and a semiconducting layer formed on the surface to come in contact with a dye solution containing the sensitizing-dye compound of the present invention and a solvent. The association of the sensitizing-dye compound and the semiconductor can be maintained through chemical bond or electrostatic interaction achieved between the anchoring group of the sensitizing-dye compound and the semiconductor material. Other methods than the adsorption for achieving association of the dye and the semiconductor would be known to a person skilled in the art.

As the electrolyte, a liquid electrolyte, a solid electrolyte, or a solution containing the electrolyte can be used. In the sense of the present invention, the electrolyte is preferably a redox electrolyte, containing a substance forming a redox system, for example a solution containing the cobalt complex described herein and/or optional iodine and imidazolium salt of iodide. As the suitable solvent for the solution, an electrochemically inert substance, such as acetonitrile or propionitrile can be used.

The DSSC can be formed, for example, by filling with the electrolytic solution between the two electrodes face to face. The two electrodes can be thus disposed with a desired distance between them by securing them by sandwiching a spacer between them. Nonetheless, other methods of manufacturing the DSSC would be well understood by a person skilled in the art.

The present invention also concerns a metal complex having the formula (I):

ML1L2X1X2 (I)

wherein M represents a metal belonging to Group 6, 8, 9, 10 or 11 of the long-format Periodic Table, preferably ruthenium; X1 and X2 are independently selected from the group consisting of halide, -OH, -CN, -SCN, -NCS, and -NCO, preferably -NCS; and L1 and L2 are independently selected from bipyridine ligands, in which at least one of L1 and L2 correspond to the formula (II) below: 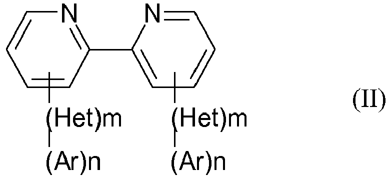 wherein Het is independently selected from a bridge selected from the group consisting of aromatic amine groups, heteroatoms, alkenyl groups, aryl groups, and heterocycles, in particular thiophene, oligothiophenes, ethylenedioxythiophenes (EDOT), oligo-EDOTs, cyclopentanedithiophenes (CPDT), and any combination thereof, preferably sulfur and thiophene, Ar is independently selected from substituted aryl groups, preferably alkoxy phenyl groups and thioalkyl phenyl groups, m is independently 1, 2, 3, or 4, and n is 1, 2, 3, or 4.

Preferably, a metal complex according to the present invention has at least one ligand of formula (II-1) below: 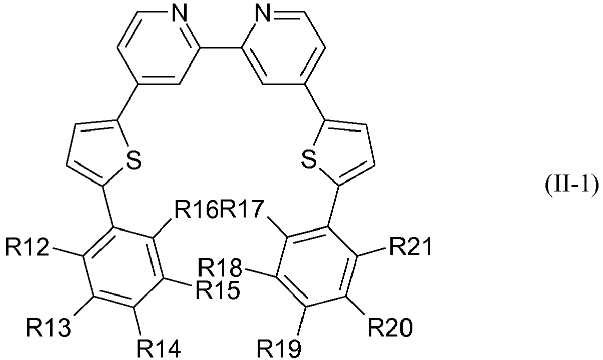 wherein R12, R13, R14, R15, R16, R17, R18, R19, R20 and R21 are independently selected from the group consisting of hydrogen, halogens, cyano, alkyl groups, halogenated alkyl groups, alkoxy groups, halogenated alkoxy groups, aryl groups, halogenated aryl groups, aryloxy groups, halogenated aryloxy groups, heterocycles, halogenated heterocycles, heterocyclic alkyl groups, halogenated heterocyclic alkyl groups, heterocyclic alkoxy groups, halogenated heterocyclic alkoxy groups, heterocyclic aryl groups, halogenated heterocyclic aryl groups, heterocyclic aryloxy groups, halogenated heterocyclic aryloxy groups, amino groups, halogenated amino groups, and NO₂. Preferably, R12, R14, R19 and R21, or R14 and R19 are alkoxy groups or thioalkyl groups, while the rest are hydrogen. Preferred examples of alkoxy groups or thioalkyl groups include butyloxy, pentyloxy, hexyloxy, heptyloxy, octyloxy, nonyloxy and decyloxy group, thiobutyl, thiopentyl, thiohexyl, thioheptyl, thiooctyl, thiononyl and thiodecyl group, especially hexyloxy group.

In a special embodiment according to the present invention, the metal complex has any one of the formulae (I-1) below: 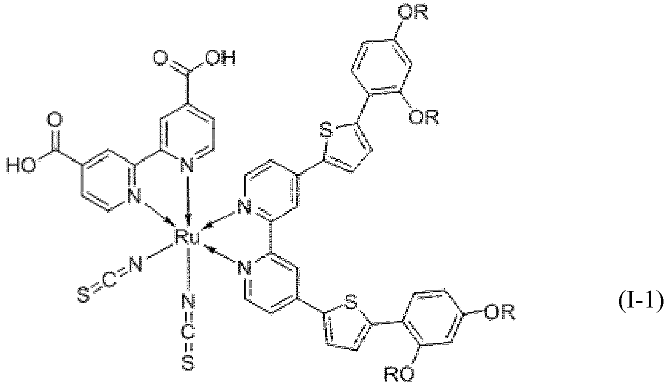 wherein R is alkyl groups, particularly hexyl group.

Further aspect of the present invention provides a ligand having the formula (II-1): 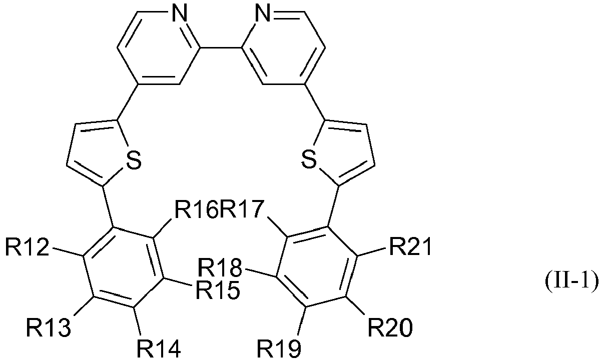 wherein R12, R13, R14, R15, R16, R17, R18, R19, R20 and R21 are independently selected from the group consisting of hydrogen, halogens, cyano, alkyl groups, halogenated alkyl groups, alkoxy groups, halogenated alkoxy groups, aryl groups, halogenated aryl groups, aryloxy groups, halogenated aryloxy groups, heterocycles, halogenated heterocycles, heterocyclic alkyl groups, halogenated heterocyclic alkyl groups, heterocyclic alkoxy groups, halogenated heterocyclic alkoxy groups, heterocyclic aryl groups, halogenated heterocyclic aryl groups, heterocyclic aryloxy groups, halogenated heterocyclic aryloxy groups, amino groups, halogenated amino groups, and NO₂. Preferably, R12, R14, R19 and R21, or R14 and R19 are alkoxy groups or thioalkyl groups, while the rest are hydrogen. Preferred examples of alkoxy groups or thioalkyl groups include butyloxy, pentyloxy, hexyloxy, heptyloxy, octyloxy, nonyloxy and decyloxy group, thiobutyl, thiopentyl, thiohexyl, thioheptyl, thiooctyl, thiononyl and thiodecyl group, especially hexyloxy group.

The metal complexes and ligands according to the present invention can be prepared by the method for manufacturing the metal complex which comprises reacting substituted aryl halide with heterocycle-boronic acid. Preferred substituted aryl halide is substituted aryl bromide, more preferably alkoxy- or thioalkyl-substituted phenyl bromide. Preferred heterocycle-boronic acid is thienyl boronic acid. Therefore, the present invention further concerns a method for manufacturing the metal complex or the ligands according to the present invention, which comprises reacting substituted aryl halide with heterocycle-boronic acid. Exemplary reaction scheme for the ligand having the formula (II-1) is as follows:

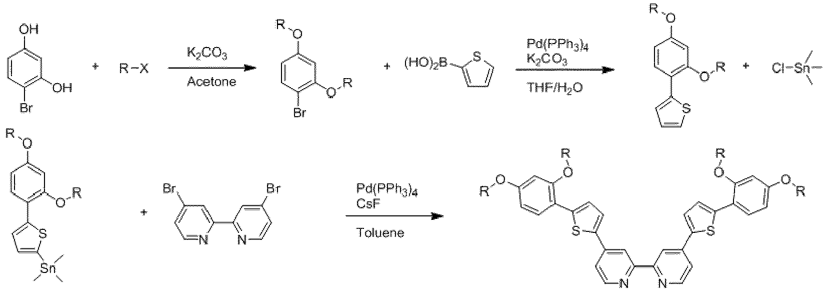

While preferred embodiments of this invention have been shown and described, modifications thereof can be made by one skilled in the art without departing from the spirit or teaching of this invention. The embodiments and examples described herein are exemplary only and are not limiting. Many variations and modifications of systems and methods are possible and are within the scope of the invention. Accordingly, the scope of protection is not limited to the embodiments described herein, but is only limited by the claims that follow, the scope of which shall include all equivalents of the subject matter of the claims.

### SHORT DESCRIPTION OF THE FIGURES

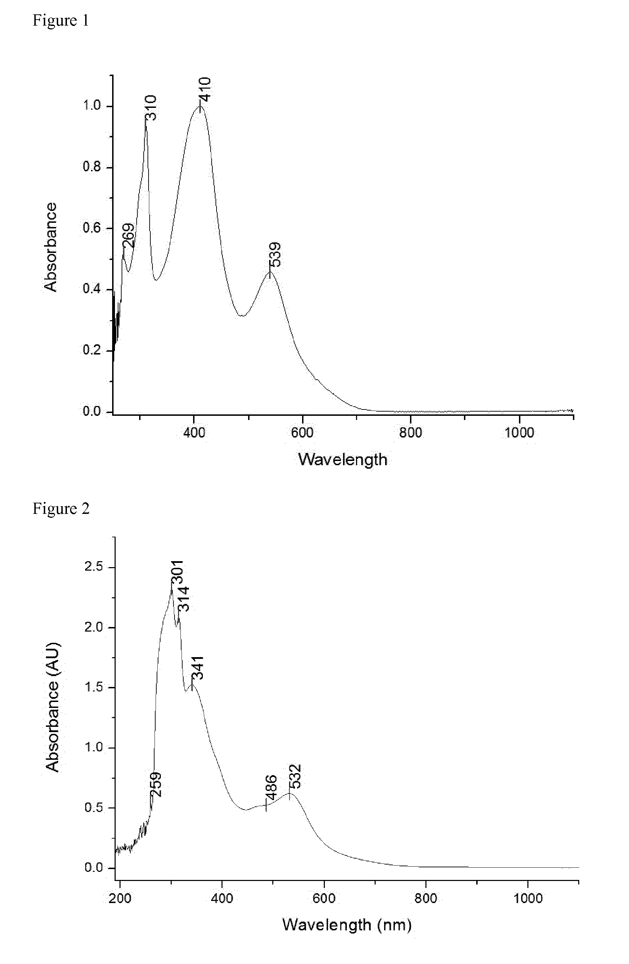

Figure 1: Normalized UV-absorption spectra for the compound of formula (I-1) in which R is hexyl group, prepared according to Example 1, measured in ethanol solution.
Figure 2: Normalized UV-absorption spectra for the compound of formula (I-2) in which R is hexyl group, prepared according to Example 2, measured in ethanolic solution.

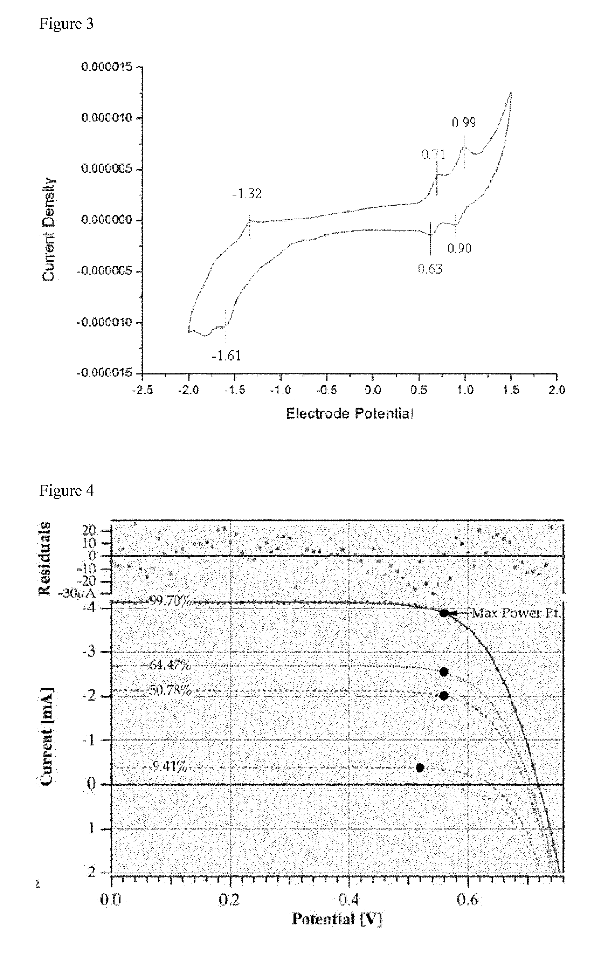

Figure 3: Cyclic voltammogram of the compound of formula (I-2) according to Example 2 in DMF at room temperature under argon with 0.1 M TBAPF₆ electrolyte, wherein the working electrode is a glassy carbon and the scan rate is 0.1 V/s.
Figure 4: Current-voltage characteristics of photovoltaic device with the compound of formula (I-1) in which R is hexyl group, prepared according to Example 3, and measured according to Example 4.

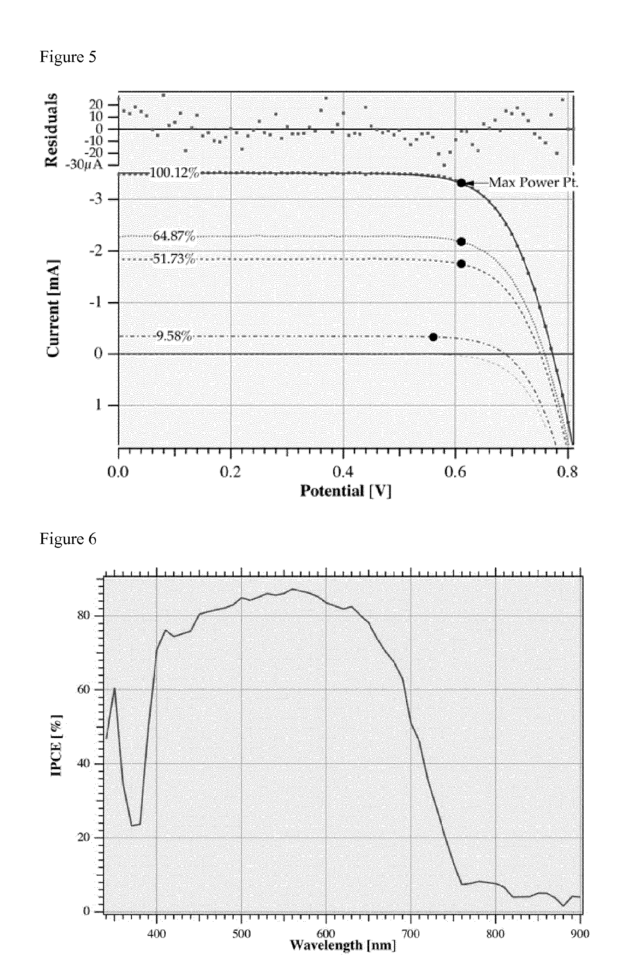

Figure 5: Current-voltage characteristics of photovoltaic device with the compound of formula (I-2) in which R is hexyl group, prepared according to Example 3, and measured according to Example 4.
Figure 6: Incident photon to current conversion efficiency plotted as a function of excitation wavelength of the compound of formula (I-1) in which R is hexyl group, prepared according to Example 3, and measured according to Example 4.

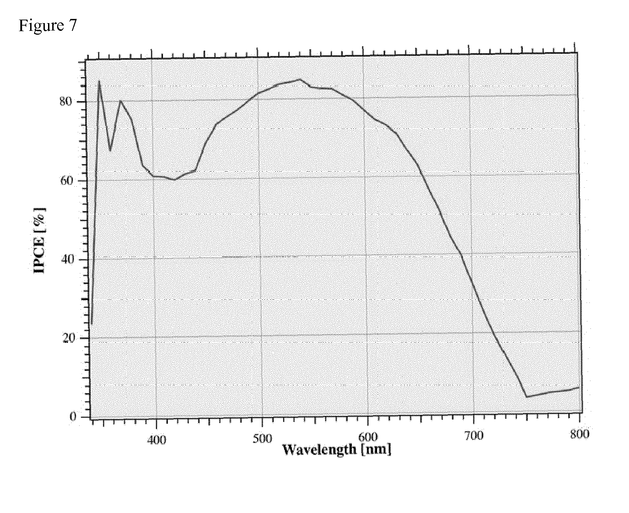

Figure 7: Incident photon to current conversion efficiency plotted as a function of excitation wavelength of the formula compound of (I-2) in which R is hexyl group, prepared according to Example 3, and measured according to Example 4.

### Examples

### Example 1: Synthesis of metal complex of the formula (I-1) in which R is hexyl

### Example 1-1: Preparation of 2-(2,4-bis(hexyloxy)phenyl)thiophene (IJL-154)

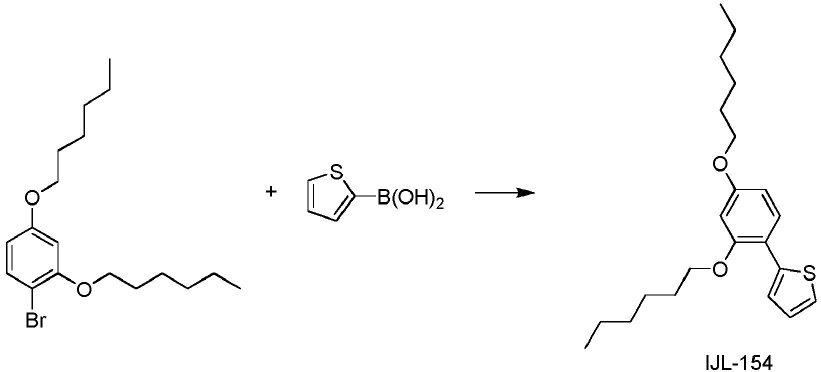

To a mixture of 1-bromo-2,4-bis(hexyloxy)benzene (2.92 g, 8.17 mmol), 2-thiopheneboronic acid (1.15 g, 8.99 mmol), sodium carbonate (1.73 g, 16.3 mmol), and Pd(PPh₃)₄ (0.47 g, 0.41 mmol) were added degassed THF (50 ml) and water (25 ml). The solution was refluxed for overnight. After cooling, water (50 ml) was added to this mixture, then organic compounds were extracted with dichloromethane (50 ml, 3 times) and then the organic phase was washed by brine (50 ml). The product was purified by column chromatography on silica gel as white solid (R_{f} = 0.2 in Hx, Yield = 2.57 g (81.9 %)) **(IJL-143)** ¹H NMR (400 MHz, CDCl₃): 7.58 (d, J= 7.6 Hz, 1H), 7.44 (dd, *J*= 3.6, 1.2 Hz, 1H), 7.28 (dd, J = 5.2, 1.2 Hz, 1H), 7.09 (dd, *J =* 5.2, 3.6 Hz, 1H), 6.57 - 6.53 (m, 2H), 4.07 (t, J= 6.8 Hz, 2H), 4.00 (t, J= 6.8 Hz, 2H), 1.93 (q, 6.8 Hz, 2H), 1.83 (q, 6.8 Hz, 2H), 1.60 ∼ 1.48 (m, 4H), 1.43 ∼ 1.36 (m, 8H), 0.98 - 0.94 (m, 6H). MS (GC-EI): *mlz:* calcd for: 360.21; found: 361.

### Example 1-2: Preparation of (5-(2,4-bis(hexyloxy)phenyl)thiophen-2-yl)trimethylstannane (IJL-159).

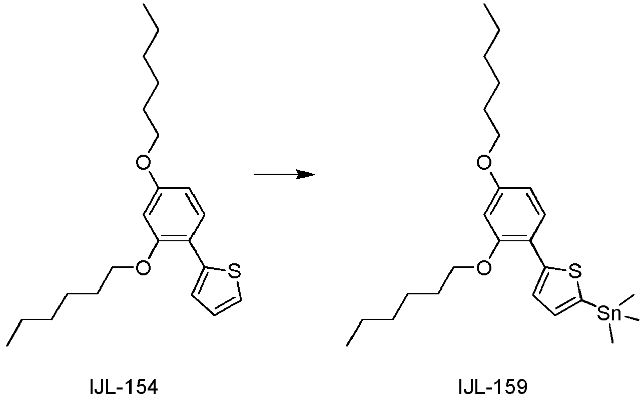

2-(2,4-bis(hexyloxy)phenyl)thiophene (IJL-154, 2.0 g, 5.55 mmol) was put into the flask and vacuum dry, and then the THF solvent (100 ml) was added. The temperature of the flask was decreased to -78°C, then dropwised the n-BuLi (1.6 M in Hx, 3.8 ml, 6.10 mmol). The temperature was slowly increased to 0°C for 1hr with stirring. The temperature was decreased to -78 °C again, and then the trimethyltin chloride (1.22 g, 6.10 mmol) was put into the flask. Stirred overnight at 20 °C. The water was put into flask of the reaction mixture after finished the reaction, and then organic phase was extracted by dichloromethane. The resultant was collected and the all organic solvent was removed. This product could be used without further purification.

### Example 1-3 : Preparation of 4,4'-bis(5-(2,4-bis(hexyloxy)phenyl)thiophen-2-yl)-2,2'-bipyridine (IJL-160).

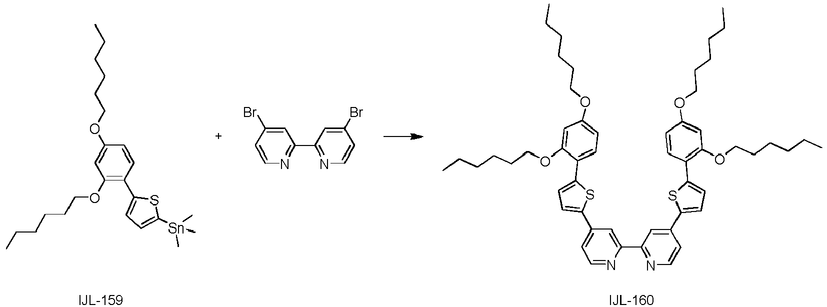

To a mixture of (5-(2,4-bis(hexyloxy)phenyl)thiophen-2-yl)trimethylstannane (IJL-159, 1.10 g, 2.10 mmol), 4,4-dibromo-2,2-bipyridyl (0.3 g, 0.96 mmol), cesium fluoride (0.58 g, 3.82 mmol), and Pd(PPh₃)₄ (0.11 g, 0.096 mmol) were added degassed toluene (100 ml). The solution was refluxed for overnight. After cooling, water (50 ml) was added to this mixture, then organic compounds were extracted with dichloromethane (50 ml, 3 times) and then the organic phase was washed by brine (50 ml). The product was purified by column chromatography on silica gel as white solide (R_{f} = 0.5 in EA : DCM = 1 : 9, Yield = 0.51 g (60.9 %))

**(IJL-160)** ¹H NMR (400 MHz, CDCl₃): 8.68 - 8.65 (m, 2H), 7.63 (d, J= 4.0 Hz, 2H), 7.61 (d, J= 9.2 Hz, 2H), 7.51 (dd, J= 5.2, 2.0 Hz, 2H), 7.46 (d, *J=* 4.0 Hz, 2H), 6.56 ∼ 6.53 (m, 4H), 4.10 (t, J= 6.4 Hz, 4H), 4.00 (t, J= 6.4 Hz, 4H), 1.95 (t, J= 6.8 Hz, 4H), 1.80 (t, J= 6.8 Hz, 4H), 1.63 ~ 1.45 (m, 8H), 1.43 ∼ 1.33 (m, 16H), 0.94 ∼ 0.89 (m, 12H). MS (GC-EI): *m*/*z:* calcd for : 872.46; found: 873.

### Example 1-4: Preparation of methylester complex of the formula (I-1) in which R is hexyl.

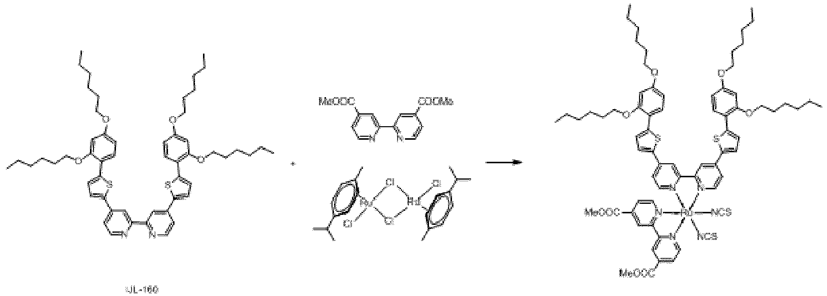

A mixture of [{RuCl₂(p-cymene)}₂](0.086 g, 0.150 mmol) and the ligand (4,4'-bis(5-(2,4-bis(hexyloxy)phenyl)thiophen-2-yl)-2,2'-bipyridine, IJL-160, 0.25 g, 0.277 mmol) was vacuum-dried and added DMF (50 ml). The reaction mixture was heated at 110 °C under nitrogen for 4 h. 2,2-bipyridyl-4,4-dicarboxylic methylester (0.078 g, 0.277 mmol) was added to the solution and refluxed at 160 °C for another 4 h under dark condition. Excess of NH₄NCS (0.138 g, 1.81 mmol) was added to the reaction mixture and heated at 130 °C for overnight. The solvent was removed using a vacuum distillation after finished the reaction. The product was purified by column chromatography on silica gel as white solide (R_{f} = 0.3 in EA: DCM = 1: 19, Yield = 0.12 g (31.8 %)).
**(methylester complex of the formula (I-1) in which R is hexyl)** ¹H NMR (400 MHz, CDCl₃): 9.77 (d, J= 6.0 Hz, 1H), 9.15 (d, J= 5.2 Hz, 1H), 8.73 (s, 1H), 8.59 (s, 1H), 8.21 (s, 1H), 8.09 (s, 1H), 8.00 ∼ 7.94 (m, 3H), 7.82 (d, J= 8.8 Hz, 1H), 774 (d, J = 7.6 Hz, 1H), 7.67 (d, J = 6.0 Hz, 2H), 7.62 (s, 1H), 7.47 (dd, *J =* 6.0, 1.2 Hz, 1H), 7.45 (s, 1H), 6.83 (s, 1H), 6.79 (s, 1H), 6.63 (dd, J = 8.4, 2.0 Hz, 1H), 6.59 (d, J = 2.4 Hz, 1H), 6.57 (dd, J = 8.8, 2.0 Hz, 1H), 6.52 (d, J = 2.0 Hz, 1H), 4.13 ∼ 3.96 (m, 8H), 4.10 (s, 3H), 3.96 (s, 3H), 2.02 - 1.76 (m, 8H), 1.55 ∼ 1.44 (m, 8H), 1.40 ∼ 1.18 (m, 16H), 0.96 - 0.90 (m, 6H), 0.87 (t, J= 7.2 Hz, 3H), 0.73 (t, J= 7.2 Hz, 3H). MS (GC-EI): *m*/*z:* calcd for : 1362.40 ; found: 1363.

### Example 1-5: Preparation of the formula (I-1) in which R is hexyl.

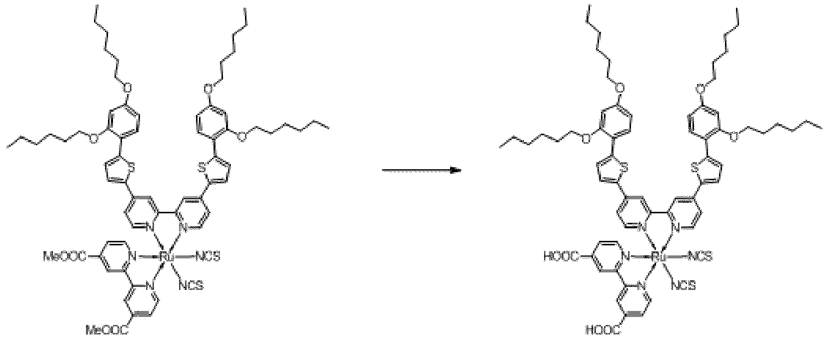

To a solution of the product obtained in Example 1-4 (0.09 g, 0.066 mmol) in acetonitrile (100 ml) was added tetrabutylammonium hydroxide (TBAOH 40% in water solution, 0.096 g, 0.166 mmol) and ammonium thiocyanate (NH₄NCS, 0.021g, 0.264 mmol), then stirred for 1hr. The reaction was checked using TLC and UV. The solvent was dried when the reaction was finished. The mixture was dissolved in water solution. Then, the mixture was acidified by 0.05M HNO₃ solution (until pH 5). A solution was left in the refrigerator. The mixture was filtered through a sintered glass crucible. (Yield = 69 mg (78.3 %)
**(the formula (I-1) in which R is hexyl)** ¹H NMR (400 MHz, DMSO): 9.71 (d, J= 6.0 Hz, 1H), 9.06 (d, J= 6.0 Hz, 1H), 8.99 (s, 1H), 8.80 (s, 1H), 8.35 (d, J= 4.8 Hz, 1H), 8.24 (s, 1H), 8.11 ∼ 8.09 (m, 2H), 7.96 - 7.82 (m, 3H), 7.73 - 7.63 (m, 3H), 7.51 (dd, J= 6.0, 1.2 Hz, 1H), 7.30 (s, 1H), 6.95 (d, J= 6.0 Hz, 1H), 6.74 - 6.52 (m, 5H), 4.14 ∼ 3.94 (m, 8H), 1.87 ∼ 1.71 (m, 8H), 1.63 ∼ 1.55 (m, 8H), 1.39 ∼ 1.31 (m, 16H), 0.97 - 0.91 (m, 6H), 0.81 (t, J= 7.2 Hz, 3H), 0.53 (t, J= 7.2 Hz, 3H). MS (GC-EI): *m*/*z:* calcd for : 1334.37 ; found: 1335.

### Example 2: Synthesis of metal complex of the formula (I-2) in which R is hexyl

### Example 2-1 : Preparation of 2-(2,4-bis(hexyloxy)phenyl)-4,4,5,5-tetramethyl-1,3,2-dioxaborolane. (IJL-142)

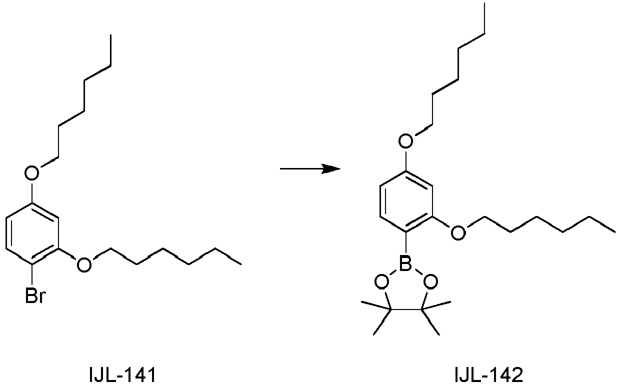

1-bromo-2,4-bis(hexyloxy)benzene (3.5 g, 9.79 mmol) was put into the flask and vacuum dried, and then the THF solvent (100 ml) was put. The temperature of the flask was decreased to -78 °C, and then dropwised the n-BuLi (1.6 M in Hx, 6.74 ml, 10.8 mmol). The temperature was slowly increased to 0°C for 1hr with stirring. The temperature was decreased to -78°C again, and then the 2-isopropoxy-4,4,5,5-tetramethyl-1,3,2-dioxaborolane (2.0 g, 10.8 mmol) was put into the flask. Stirred overnight at 20 °C. The water was put into flask of the reaction mixture after finished the reaction, and then organic phase was extracted by dichloromethane. The resultant was collected and the all organic solvent was removed. The product was purified by column chromatography on silica gel as colorless powder (R_{f} = 0.5 in EA : Hx = 1 : 19, Yield = 3.20 g (80.8 %)). (IJL-142) ¹H NMR (400 MHz, CDCl₃): 7.58 (d, J= 7.6 Hz, 1H), 6.45 (dd, J= 7.6, 2.0 Hz, 1H), 6.39 (d, J= 2.0 Hz, 1H), 3.97 ∼ 3.92 (m, 4H), 1.80 ~ 1.75 (m, 4H), 1.56 ~ 1.43 (m, 4H), 1.37 ~ 1.32 (m, 8H), 0.93 ~ 0.89 (m, 6H). MS (GC-EI): m/z: calcd for C₂₄H₄₁BO₄: 404.31; found: 405.

### Example 2-2: Preparation of 4,4'-bis(2,4-bis(hexyloxy)phenyl)-2,2'-bipyridine (IJL-143).

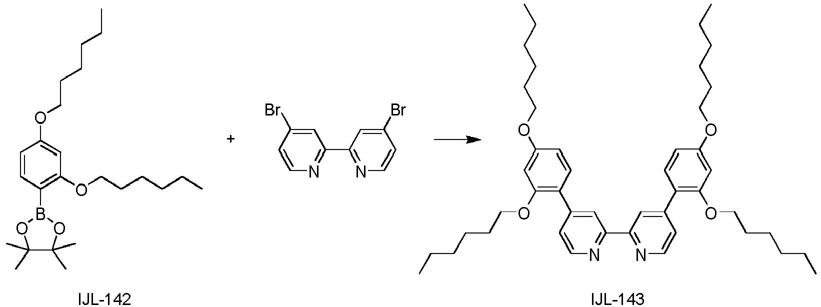

To a mixture of 2-(2,4-bis(hexyloxy)phenyl)-4,4,5,5-tetramethyl-1,3,2-dioxaborolane (IJL-142, 2.6 g, 6.43 mmol), 4,4-dibromo-2,2-bipyridyl (0.8 g, 2.57 mmol), potassium carbonate (1.42 g, 10.29 mmol), and Pd(PPh₃)₄ (0.15 g, 0.129 mmol) were added degassed THF (100 ml) and water (50 ml). The solution was refluxed for overnight. After cooling, water (50 ml) was added to this mixture, then organic compounds were extracted with dichloromethane (50 ml, 3 times) and then the organic phase was washed by brine (50 ml). The product was purified by column chromatography on silica gel as white solid (R_{f} = 0.2 in EA : Hx = 1 : 4, Yield = 1.43 g (78.5 %)).

**(IJL-143)** ¹H NMR (400 MHz, CDCl₃): 8.64 (dd, J = 5.2, 0.8 Hz, 2H), 8.59 (dd, J= 1.6, 0.8 Hz, 2H), 7.54 (dd, J= 5.2, 2.0 Hz, 2H), 7.43 (d, J = 8.4 Hz, 2H), 6.59 - 6.56 (m, 4H), 4.02 - 3.96 (m, 8H), 1.83 ∼ 1.73 (m, 8H) 1.51 ∼ 1.34 (m, 16H), 1.27 ∼ 1.23 (m, 8H), 0.94 - 0.91 (m, 6H), 0.84 - 0.81 (m, 6H). MS (GC-EI): *m*/*z:* calcd for: 708.49; found: 709.

### Example 2-3: Preparation of the formula (1-2) in which R is hexyl.

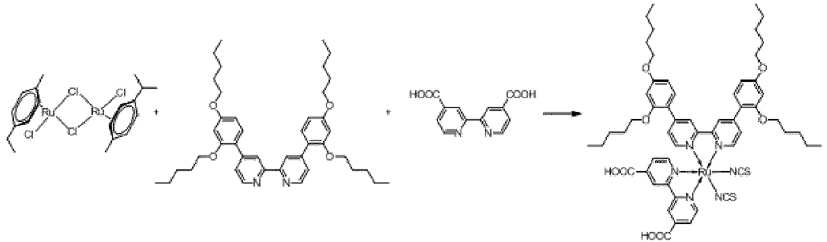

A mixture of [{RuCl₂(p-cymene)}₂] (0.138 g, 0.241 mmol) and the ligand (4,4'-bis(2,4-bis(hexyloxy)phenyl)-2,2'-bipyridine, IJL-143, 0.3 g, 0.459 mmol) was vacuum-dried and added DMF (50 ml). The reaction mixture was heated at 110 °C under nitrogen for 4 h. 2,2-Bipyridyl-4,4-dicarboxylic acid (0.112 g, 0.459 mmol) was added to the solution. refluxed at 160 °C for another 4 h under dark condition. Excess of NH₄NCS (0.22 g, 2.89 mmol) was added to the reaction mixture and heated at 130 °C for overnight. The solvent was removed using a vacuum distillation after finished the reaction. Water was added to the resulting semisolid to remove excess NH₄NCS. The water-insoluble product was collected on a sintered glass crucible by suction filtration and washed with distilled water and diethylether. The crude complex was dissolved in methanol. Sephadex LH-20 (2×30 cm) column and eluted with methanol. (Yield = 0.18 g (39.2 %))
**(the formula (1-2) in which R is hexyl)** ¹H NMR (400 MHz, MeOD): 9.71 (d, J= 6.0 Hz, 1H), 9.31 (d, J= 5.6 Hz, 1H), 9.05 (s, 1H), 8.89 (s, 1H), 8.77 (dd, J= 4.4, 1.2 Hz, 1H), 8.58 (s, 1H), 8.31 (d, *J* = 6.4 Hz, 1H), 7.90 (d, J = 6.4 Hz, 1H), 7.72 (dd, J = 8.0, 1.6 Hz, 1H), 7.67 - 7.64 (m, 1H), 7.40 (d, J = 7.2 Hz, 1H), 7.38 - 7.36 (m, 1H), 7.30 (d, J= 6.4 Hz, 1H), 6.77 - 6.59 (m, 5H), 4.12 ∼ 3.96 (m, 8H), 1.87 ∼ 1.62 (m, 8H), 1.45 ∼ 1.27 (m, 16H), 1.14 ∼ 1.00 (m, 8H), 0.96 ∼ 0.90 (m, 6H), 0.71 ∼ 0.62 (m, 6H). MS (GC-EI): *m*/*z:* calcd for : 1114.33 ; found: 1115.

### Example 3: Fabrication of Dye-Sensitized Solar Cells

The FTO glass substrates were immersed in 40 mM TiCl₄ aq. at 70 °C for 30 min and washed with water and ethanol. The 8 µm thick mesoporous nano-TiO₂ films composed of 20 nm anatase TiO₂ particles were coated on the FTO glass plates by repetitive screen printing. After drying the nanocrystalline TiO2 layer at 125 °C, a 4 µm thick second layer of 400 nm sized light scattering anatase particles (CCIC, HPW-400) was deposited by screen printing onto the transparent layer. The TiO₂ electrodes were gradually heated under an air flow at 325 °C for 5 min, at 375 °C for 5 min, at 450 °C for 15 min and 500 °C for 15 min. The TiO₂ electrodes were treated again by TiCl4 and sintered at 500 °C for 30 min. The dye-adsorbed TiO₂ electrode and thermally platinized counter electrode were assembled into a sealed sandwich type cell with a gap of a hotmelt ionomer film (Surlyn 1702, 25 µm thickness, Du-Pont) as a spacer between the electrodes.

A drop of electrolyte solution was placed on the drilled hole in the counter electrode of the assembled cell and was driven into the cell via vacuum backfilling, then the hole was sealed using additional Surlyn and a cover glass.

### Example 4: Evaluation of Dye-Sensitized Solar Cells

The measurement of incident photon-to-current conversion efficiency (IPCE) was plotted as a function of excitation wavelength by using the incident light from a 300 W xenon lamp (ILC Technology, USA), which was focused through a Gemini-180 double monochromator (Jobin Yvon Ltd.). In order to reduce scattered light from the edge of the glass electrodes of the dyed TiO₂ layer, a light shading mask was used onto the DSSCs, so active area of DSSCs was fixed to 0.22 cm². The irradiation source was a 450 W xenon light source (Osram XBO 450, USA), whose power of an AM 1.5 solar simulator was calibrated by using a reference Si photodiode equipped with an IR cutoff filter (KG-3, Schott) in order to reduce the mismatch in the region of 350-750 nm between the simulated light and AM 1.5 to less than 2%. The measurement delay time of photo I-V characteristics of DSSCs was fixed to 40 ms.

**Table 1: Measurements for the formula (I-1) complex according to Example 1.**

| Dye | Electrolyte | Thickness of TiO2 (mm) | Jsc (mA/cm2) | Voc (mV) | η (%) |
|---|---|---|---|---|---|
| Formula (I-1) | Iodine | 8+4 | 18.87 | 719 | 9.9 |
| Formula (I-1) | Co(bpy)+2/+3 | 4+4 | 10.7 | 804 | 6.26 |

**Table 2: Measurements for the formula (I-2) complex according to Example 2.**

| Dye | Electrolyte | Thickness of TiO2 (mm) | Jsc (mA/cm2) | Voc (mV) | η (%) |
|---|---|---|---|---|---|
| Formula (I-2) | Iodine | 8+4 | 15.85 | 772 | 9.2 |
| Formula (I-2) | Co(bpy)+2/+3 | 4+4 | 11.34 | 825 | 6.96 |

## Claims

1. A dye-sensitized solar cells, comprising a metal complex comprising at least one substituted monopyridine ligand or polypyridine ligand, as a sensitizing-dye, and a cobalt complex, as a redox mediator, wherein the substituted monopyridine ligand and/or polypyridine ligand comprises at least one substituted aryl group.

2. The dye-sensitized solar cell according to claim 1, wherein the substituted monopyridine ligand or polypyridine ligand comprises any one of the structures below:

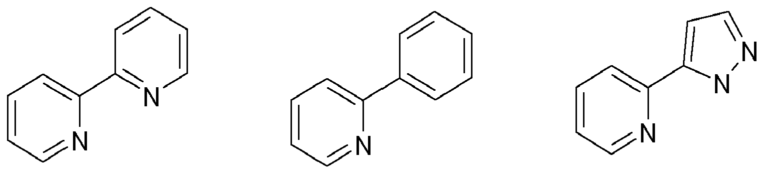

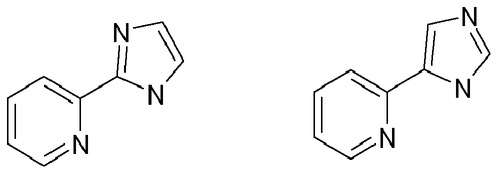

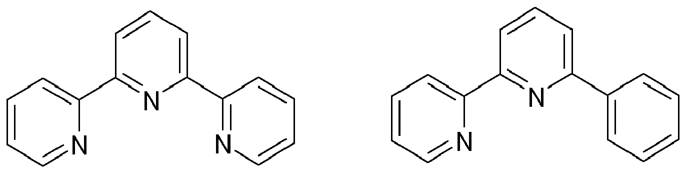

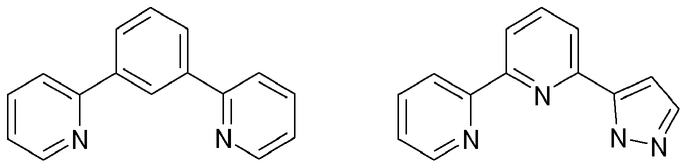

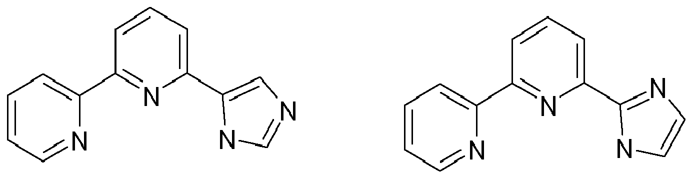

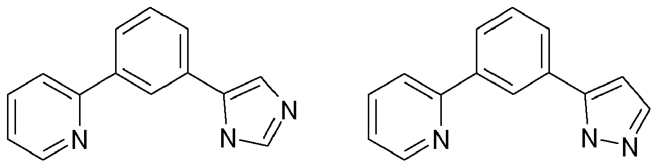

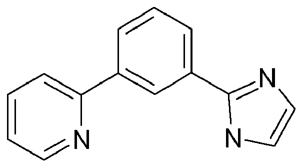

3. The dye-sensitized solar cell according to claim 1 or 2, wherein the substituted aryl group is selected from alkoxy or thioalkyl phenyl groups.

4. The dye-sensitized solar cell according to any one of claims 1 to 3, wherein the substituted aryl group is connected to the substituted monopyridine ligand and/or polypyridine ligand through a bridge selected from the group consisting of aromatic amine groups, heteroatoms, alkenyl groups, aryl groups, and heterocycles, in particular thiophene, oligothiophenes, ethylenedioxythiophenes (EDOT), oligo-EDOTs, cyclopentanedithiophenes (CPDT), and any combination thereof, preferably sulfur atom and thiophene.

5. The dye-sensitized solar cell according to any one of claims 1 to 4, wherein the cobalt complex has the formula (A) :
[L¹ₐCoL²_{b}](X_{c}) (A)
wherein Co is cobalt; a and b are independently integer from 0 to 6, c is integer from 0 to 3 ;
each X is independently a counter-anion, preferably selected from borates or phosphates ;
each L¹ is independently selected from the group consisting of unsubstituted or substituted bipyridine ligands and unsubstituted or substituted terpyridine ligands ; and
each L² is independently a co-ligand, preferably selected from the group consisting of Cl-, Br-, I-, CN-, NCO-, NCS-, NCSe-, amino groups, phosphate groups, ClO₄⁻, PF₆⁻, borates such as [BF₄]⁻, tetracyanoborate ([B(CN)₄]⁻), [B(Ph)₄]⁻, [B(C₆F₅)₄]⁻, bis(trifluoromethanesulfonyl)imide (TFSI⁻), bis(fluorosulfonyl)imide (FSI⁻), and any combination thereof, more preferably ClO₄⁻, PF₆⁻, [BF₄]⁻, ([B(CN)₄]⁻), TFSI⁻ or FSI⁻.

6. The dye-sensitized solar cell according to any one of claims 1 to 5, wherein L¹ is independently selected from the group consisting of the following ligands and a is 2 or 3. 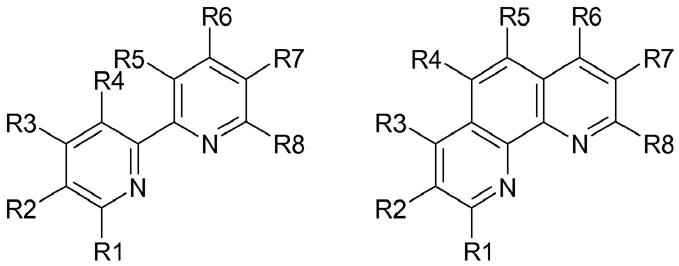 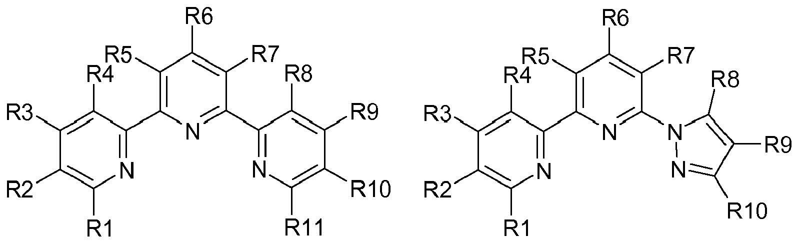 wherein R1, R2, R3, R4, R5, R6, R7, R8, R9, R10 and R11 are independently selected from the group consisting of hydrogen, halogens, in particular fluorine, amino groups, NO₂, CN, alkyl groups, halogenated alkyl groups, aryl groups, halogenated aryl groups, particularly hydrogen, fluorine, alkyl groups, and fluorinated alkyl groups.

7. The dye-sensitized solar cell according to any one of claims 1 to 6, wherein the metal complex has the formula (I) :
ML1L2X1X2 (I)
wherein M represents a metal belonging to Group 6, 8, 9, 10 or 11 of the long-format Periodic Table, preferably ruthenium ;
X1 and X2 is independently selected from the group consisting of halide, -OH, - CN, -SCN, -NCS, and -NCO; and
L1 and L2 are independently selected from bipyridine ligands, in which at least one of L1 and L2 correspond to the formula (B) or the formula (II) below : 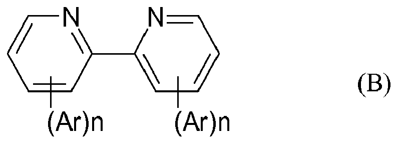 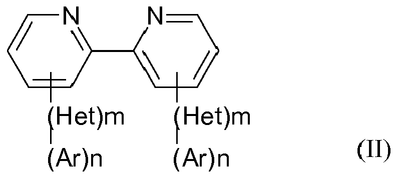 wherein Het is independently selected from a bridge selected from the group consisting of aromatic amine groups, heteroatoms, alkenyl groups, aryl groups, and heterocycles, in particular thiophene, oligothiophenes, ethylenedioxythiophenes (EDOT), oligo-EDOTs, cyclopentanedithiophenes (CPDT), and any combination thereof, preferably sulfur and thiophene, Ar is independently selected from substituted aryl groups, preferably alkoxy phenyl groups and thioalkyl phenyl groups, m is independently 1, 2, 3, or 4, and n is 1, 2, 3, or 4.

8. The dye-sensitized solar cell according to claim 7, wherein L1 has the formula (B) or the formula (II) and L2 has the formula (III) below : 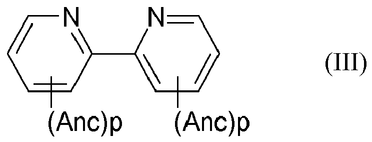 wherein Anc is independently selected from anchoring groups, and p is independently 1, 2, 3 or 4, preferably 1.

9. The dye-sensitized solar cell according to any one of claims 1 to 8,
wherein the anchoring group is selected from the group consisting of -COOH, -PO₃H₂, -P(=O)(Ra)(OH) (wherein Ra is selected from the groups consisting of optionally branched C1-C18 alkyl groups and optionally substituted C5-C12 aryl groups, and halogenated derivatives thereof), -PO₄H₂, -PO₂HRb (wherein Rb is selected from alkyl groups or aryl groups), -SO₃H, -CONHOH, -NO₂, acetylacetonate, acrylic acid derivatives, malonic acid derivative, rhodanine-3-acetic acid, propionic acid, deprotonated forms of the aforementioned, salts of said deprotonated forms, and chelating groups with π-conducting character, in particular from the group consisting of -COOH, and salts of the deprotonated forms of the aforementioned.

10. The dye-sensitized solar cell according to any one of claims 1 to 9, wherein the metal complex has at least one ligand of formula (II-1) below: 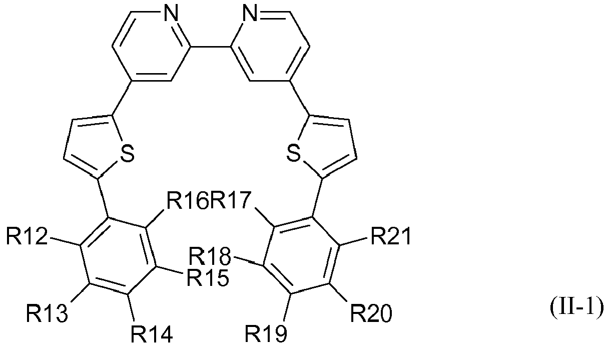 wherein R12, R13, R14, R15, R16, R17, R18, R19, R20 and R21 are independently selected from the group consisting of hydrogen, halogens, cyano, alkyl groups, halogenated alkyl groups, alkoxy groups, halogenated alkoxy groups, aryl groups, halogenated aryl groups, aryloxy groups, halogenated aryloxy groups, heterocycles, halogenated heterocycles, heterocyclic alkyl groups, halogenated heterocyclic alkyl groups, heterocyclic alkoxy groups, halogenated heterocyclic alkoxy groups, heterocyclic aryl groups, halogenated heterocyclic aryl groups, heterocyclic aryloxy groups, halogenated heterocyclic aryloxy groups, amino groups, halogenated amino groups, and NO₂, preferably alkoxy groups or thioalkyl groups.

11. A metal complex having the formula (I):
ML1L2X1X2 (I)
wherein M represents a metal belonging to Group 6, 8, 9, 10 or 11 of the long-format Periodic Table, preferably ruthenium ;
X1 and X2 is independently selected from the group consisting of halide, -OH, - CN, -SCN, -NCS, and -NCO; and
L1 and L2 are independently selected from bipyridine ligands, in which at least one of L1 and L2 correspond to the formula (II) below: 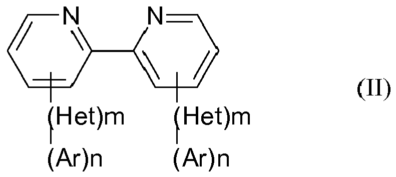 wherein Het is independently selected from a bridge selected from the group consisting of aromatic amine groups, heteroatoms, alkenyl groups, aryl groups, and heterocycles, in particular thiophene, oligothiophenes, ethylenedioxythiophenes (EDOT), oligo-EDOTs, cyclopentanedithiophenes (CPDT), and any combination thereof, preferably sulfur and thiophene, Ar is independently selected from substituted aryl groups, preferably alkoxy phenyl groups and thioalkyl phenyl groups, m is independently 1, 2, 3, or 4, and n is 1, 2, 3, or 4.

12. The metal complex according to claim 11, having at least one ligand of formula (II-1) below: 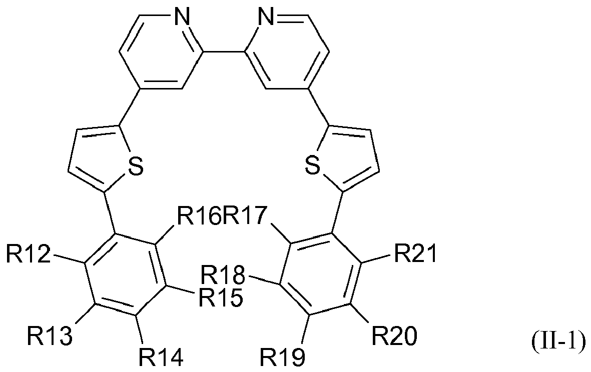 wherein R12, R13, R14, R15, R16, R17, R18, R19, R20 and R21 are independently selected from the group consisting of hydrogen, halogens, cyano, alkyl groups, halogenated alkyl groups, alkoxy groups, halogenated alkoxy groups, aryl groups, halogenated aryl groups, aryloxy groups, halogenated aryloxy groups, heterocycles, halogenated heterocycles, heterocyclic alkyl groups, halogenated heterocyclic alkyl groups, heterocyclic alkoxy groups, halogenated heterocyclic alkoxy groups, heterocyclic aryl groups, halogenated heterocyclic aryl groups, heterocyclic aryloxy groups, halogenated heterocyclic aryloxy groups, amino groups, halogenated amino groups, and NO₂, preferably alkoxy groups or thioalkyl groups.

13. The metal complex according to claim 11 or 12, having the formulae (I-1) below: 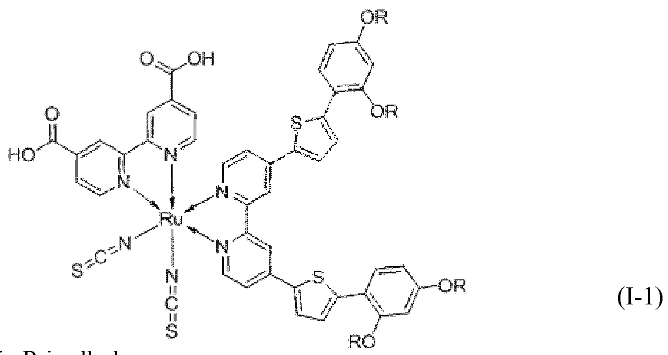 wherein R is alkyl groups.

14. A method for manufacturing the metal complex according to any one of claims 11 to 13, which comprises reacting substituted aryl halide, preferably alkoxy- or thioalkyl-substituted phenyl bromide, with heterocycle-boronic acid, preferably thienyl boronic acid.

15. A ligand having the formula (II-1): 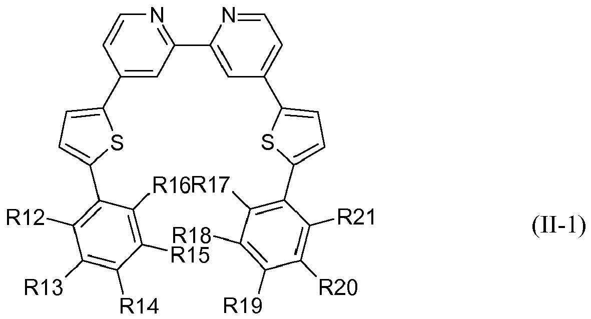 wherein R12, R13, R14, R15, R16, R17, R18, R19, R20 and R21 are independently selected from the group consisting of hydrogen, halogens, cyano, alkyl groups, halogenated alkyl groups, alkoxy groups, halogenated alkoxy groups, aryl groups, halogenated aryl groups, aryloxy groups, halogenated aryloxy groups, heterocycles, halogenated heterocycles, heterocyclic alkyl groups, halogenated heterocyclic alkyl groups, heterocyclic alkoxy groups, halogenated heterocyclic alkoxy groups, heterocyclic aryl groups, halogenated heterocyclic aryl groups, heterocyclic aryloxy groups, halogenated heterocyclic aryloxy groups, amino groups, halogenated amino groups, and NO₂, preferably alkoxy groups or thioalkyl groups.
